# EUROPEAN PATENT APPLICATION

(11) **EP 2 966 093 A1**
(43) Date of publication of application: **13.01.2016**
(21) Application number: 14175925.8
(22) Date of filing: 07.07.2014
(51) Int. Cl.: C08B 5/14, C12N 7/00

(54) **Process for the preparation of magnetic sulfated cellulose particles, magnetic sulfated cellulose particles and their use**

(71) Applicant: Max-Planck-Gesellschaft zur Förderung der Wissenschaften e.V., 80539 München (DE)
(72) Inventor: Wolff, Michael, Dr., 39175 Biederitz (DE); Pieler, Michael Martin, 39104 Magdeburg (DE); Reichl, Udo, 39104 Magdeburg (DE)
(74) Representative: Kröncke, Rolf

(57) **Abstract**

In a first aspect, the present invention relates to a process for the preparation of magnetic sulfated cellulose particles. In particular, the present invention relates to the preparation of magnetic sulfated cellulose particles under specific reaction conditions allowing providing said magnetic sulfated cellulose particles being particularly useful in further process steps including enrichment of proteinaceous compositions. In another aspect, the present invention relates to the magnetic sulfated cellulose particles itself as well as its use for isolation of proteinaceous compositions including composition of virus proteins and/or non-virus proteins as well as whole virus and for enrichment steps in *in vitro* diagnostic methods. Finally, the present invention provides a method for isolating whole virus or virus proteins as well as isolating heparin binding molecules comprising the step of affinity purification using the magnetic sulfated cellulose particles according to the present invention. The present invention relates further to a bioreactor system comprising a bioreactor and a magnetic separation unit useful for separating magnetic sulfated cellulose particles according to the present invention.

## Description

In a first aspect, the present invention relates to a process for the preparation of magnetic sulfated cellulose particles. In particular, the present invention relates to the preparation of magnetic sulfated cellulose particles under specific reaction conditions allowing providing said magnetic sulfated cellulose particles being particularly useful in further process steps including enrichment of proteinaceous compositions. In another aspect, the present invention relates to the magnetic sulfated cellulose particles itself as well as its use for isolation of proteinaceous compositions including composition of virus proteins and/or non-virus proteins as well as whole virus and for enrichment steps in *in vitro* diagnostic methods. Finally, the present invention provides a method for isolating whole virus or virus proteins as well as isolating heparin binding molecules comprising the step of affinity purification using the magnetic sulfated cellulose particles according to the present invention. The present invention relates further to a bioreactor system comprising a bioreactor and a magnetic separation unit useful for separating magnetic sulfated cellulose particles according to the present invention.

### Technological background

The isolation of proteinaceous compositions including isolation of whole virus and viral proteins plays an important role in various purification methods, for example, for the production of vaccines useful as prophylactic or therapeutic vaccines against viral infections. The vaccines include typically whole virus as well as nanoparticles containing antigens or freely suspended proteins and virus proteins. Further, isolation of non-infectious or infectious viruses or proteinaceous compositions including yeast cell derived, insect cell derived, plant cell derived or recombinantly produced proteins may be useful for *in vitro* diagnostics, the monitoring of infectious virus outbreaks and in general microbiological analysis including virological analysis.

Typically, immune responses elicited by virus vaccines are based on raising an appropriate cellular and/or humoral immunity against immunogens of the virus. Virus vaccines are based either on whole virus which may be inactivated or are based on parts of said virus including viral proteins. Typical examples for vaccines are vaccines for influenza virus including the envelope proteins of surface proteins like neuraminidase, hemagglutinin or other candidate proteins. For HIV, gp120 and gp41 are assumed to represent vaccine candidates.

In the past, numerous methods like gradient centrifugation, tangential-flow filtration, and diafiltration as well as size exclusion chromatography have been described for purification of virus proteins including whole virus particles.

Until today, conventional vaccine production processes include virus production using embryonated hen's egg (for influenza virus), primary cell lines (e.g. for vaccinia virus) or diploid cell lines (e.g. rabies virus), as well as continuous cell line cultures. For example, for smallpox vaccine production the method of choice is still primary cultured chicken embryo fibroblasts requiring downstream processing strategies allowing isolation and purification of the whole virus or virus components, in particular, of viral membrane proteins which are the main immunogens of the virus.

Various approaches have been suggested for purification of whole virus and virus proteins as mentioned above. For example, WO 96/15231 provides a general overview of methods useful for producing biologicals in protein free cultures. Said methods comprise pseudo-affinity purification steps as well as other chromatography methods, enzyme treatment steps, ultra-filtration steps and hydrophobic interaction steps.

EP 171 086 relates to a method for purification of influenza virus. The method described therein comprises subjecting a solution containing the influenza virus to column chromatography using, as a stationary chromatography phase, a sulfuric acid ester of cellulose or a cross-linked polysaccharide. Therein, the modification and functionalization of a chromatography matrix useful for pseudo-affinity column chromatography of influenza virus is described. The matrix is a sulfuric acid ester of cellulose or a cross-linked polysaccharide. The steps for functionalization of the cellulose comprises reacting pyridine with chloro-sulfonic acid at a temperature below 0°C, thereafter, heating said mixture to a temperature of about 65°C to 70°C until all crystals are dissolved and cooling the said mixture to a temperature equal or above to 30°C. Thereafter the matrix is added and maintained under constant mixing at the said temperature for 3 to 16 hours. Subsequently, the mixture is neutralized and a matrix is obtained useful for column chromatography. The product described in EP 171 086 are commercially available as Cellufine^{™} sulfate. For example, a use of said Cellufine^{™} sulfate containing sulfated cellulose beads have been applied in vaccine production processes as pseudo-affinity chromatographic matrix, see EP 0 870 508, EP 171 086, WO 2007/080123, WO 2008/006780. These cellulose beads contain low concentration of sulfate esters, the charge density of Cellufine^{™} sulfate is lower compared to commonly used ion exchangers. Further, cellulose beads generate a higher back pressure and exhibit diffusion limitations compared to cellulose membranes, which represent the main disadvantage of beads. In addition, due to the limited pressure stability of Cellufine^{™} sulfate under operating conditions (<2 bar) the flow rate is restricted resulting in sub-optimal productivity. To overcome these drawbacks, various membrane absorbers have been described.

A new type of sulfated membrane absorbers is disclosed in WO 2008/125361 relating to a method for the preparation of sulfated cellulose membranes. As demonstrated therein and in subsequent publications from the inventors, the sulfated cellulose membranes disclosed are superior over Cellufine^{™} sulfate membranes and beads in that contaminants, in particular, host cell DNA and proteins, are diminished. That is, one of the major disadvantages of most of the purification methods described in that is that these methods are limited with respect to depletion of contaminants, in particular, host cell DNA and proteins. This requires additional steps including a nuclease treatment step for host cell depletion which increases costs and is economically not desired. In addition, due to the reduced specificity of ion exchangers compared to affinity matrices, the dynamic binding capacity for the target molecules is commonly higher.

Further, major drawbacks of primary column chromatography with bead based cellulose columns include long process times with large recovery liquid volume. Flow rates of the column chromatography are limited and preparation of the columns is time consuming and cumbersome and requires regeneration steps.

Today, current regulations require very low levels of contaminants in various biological products, in particular on residual DNA levels. For biopharmaceutical product solutions used e.g. for injection including viral vaccines, the contaminants including the DNA levels should be as low as possible to reduce the risk of cellular transformations by potential oncogenic DNA and the potential of contamination with infectious DNA, e.g. for influenza vaccines, these products should contain < 10 ng cellular DNA per dose.

Hence, clearance of host cell DNA as well as new possibilities to allow high yield production of whole virus or viral particles as well as proteinaceous proteins in general, is required. In particular, in view of the further developments on cultivation and production of virus particles, like the use of cell lines, new downstream processing steps of said cultures are required. In particular, for allowing feasible viral purification in biotechnological based processes of whole virus and viral proteins, new downstream processes are required for separation and purification of the whole virus or virus particles.

Recently Sakudo et al. developed a new method using magnetic particle to facilitate the isolation of infectious viruses [1]. Therein the so called "viro-adem beads" of ademtech are used to capture human influenza A and B viruses from various liquids allowing sampling the same. The beads used therein are anionic magnetic beads based on the polymer poly(methyl vinyl ether-maleic anhydride) (poly(MVE-MA)). A major drawback of using this type of anionic polymers is the fact that due to the negative charge, all components present in the fluid being charged positively bind to these anionic beads, hence, various compounds are enriched but the enrichment of virus particles is very unspecific. In particular, host cell DNA will also bind to the anionic resin. Thus, the anionic beads may allow concentrating the viruses but do not allow separation and purification. In addition, as identified in the discussion part of said document, envelope proteins, e.g. stemming from Hepatitis B virus (HBV) could not be captured by these anionic magnetic beads. In addition, it is identified in the literature that attention should be paid to non-specific binding to other components, such as albumin or nucleic acids [2]. Hence, the anionic polymer coated beads useful for capture of human influenza A and B virus have major drawbacks.

Another approach to separate virus or viral proteins from liquids including culture medium is the use of beads coated with antibodies directed against the specific virus. However, a major drawback of such beads is the specificity of the antibodies since the antibodies are specific for one specific type of virus, namely, for one specific epitope of an antigen, only, thus, this system may not allow to use the same beads for purification of different types of virus, although, cross-reaction with other strains or types of virus may occur.

Therefore, it is an object of the present invention to provide a process for the preparation of magnetic particles useful for the isolation and purification of whole virus or virus proteins as well as methods for the purification of the whole virus or virus proteins in a simple manner overcoming the drawbacks in the art.

It is another object of the present invention to provide magnetic particles useful in the purification of proteinaceous fragments including whole virus, namely, to provide particles useful for pseudo-affinity purification of virus and viral proteins.

### Summary of the present invention

In a first aspect, the present invention relates to a process for the preparation of magnetic sulfated cellulose particles comprising the steps of providing a mixture of chloro-sulfonic acid and pyridine; adding magnetic cellulose particle to said mixture and reacting the same, e.g. at a temperature of 80°C or lower, to obtain the magnetic sulfated cellulose particles.

Particularly preferred, the magnetic cellulose particles are particles containing inorganic particles, like inorganic nanoparticles.

In another aspect, the present invention relates to the magnetic sulfate cellulose particles obtainable with a method according to the present invention. Further, the present invention relates to the use of the magnetic sulfated cellulose particles according to the present invention for isolating proteinaceous compositions, in particular, whole virus and/or viral proteins.

In another aspect, the present invention provides a method for isolating whole virus or virus proteins as well as for isolating heparin binding molecules comprising the step of pseudo-affinity purification using the magnetic sulfated cellulose particles according to the present invention.

Finally, the present invention provides a bioreactor system comprising a bioreactor and a magnetic separation unit. The magnetic separation unit allows to separate magnetically magnetic sulphated cellulose particles according to the present invention optionally having viral components, including whole virus bound from culture medium or other liquids.

### Brief description of the drawings

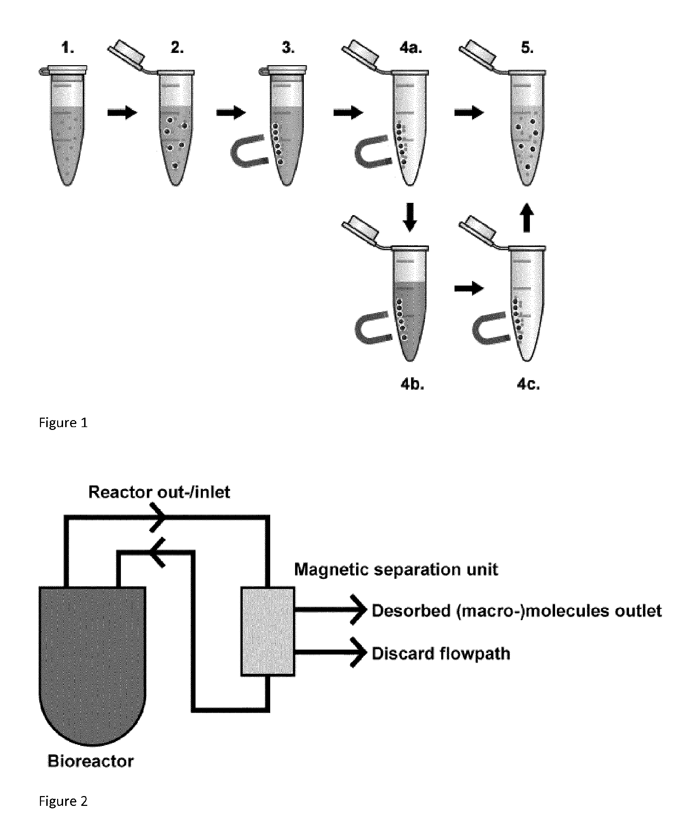

Figure 1 is a chart showing the steps of the process according to the present invention. Step 1: Shows the initial solution with (macro-)molecules of interest. Step 2: Magnetic sulfated cellulose particles (MSCP) are added to the initial solution (black/white). Step 3: MSCP with (macro-)molecules are drawn to the wall of the reaction vessel with a magnet. Step 4a: Supernatant is removed from the reaction vessel. Step 4b: Optionally washing step with buffer. Step 4c: Optionally supernatant removal step (same as step 4a). Step 5: Desorption of the (macro-)molecules of interest from the MSCP with desorption buffer. Step 1-4a and 5 show the general flow scheme. Steps 4b and 4c are optionally washing steps which can be integrated if needed.
Figure 2: figure 2 shows a scheme of a bioreactor according to the present invention. The solution in the bioreactor contains (macro-)molecule that can bind to the magnetic sulfated cellulose particles (MSCP). The magnetic sulfated cellulose particles can be applied inside the reactor to bind the (macro-)molecules of interest or can be applied after the bioreactor solution is transferred from the bioreactor to the magnetic separation unit over reactor out-/inlet connections. In the magnetic separation unit the MSCP are removed from the solution stream and the solution without the MSCP flows back into the bioreactor or is discarded. In the magnetic separation unit the (macro-)molecules are treated with the desorption puffer to remove them from the MSCP and are available from the desorbed (macro-)molecules outlet. The MSCP can be optionally recycled and used in another bind- and desorption step.

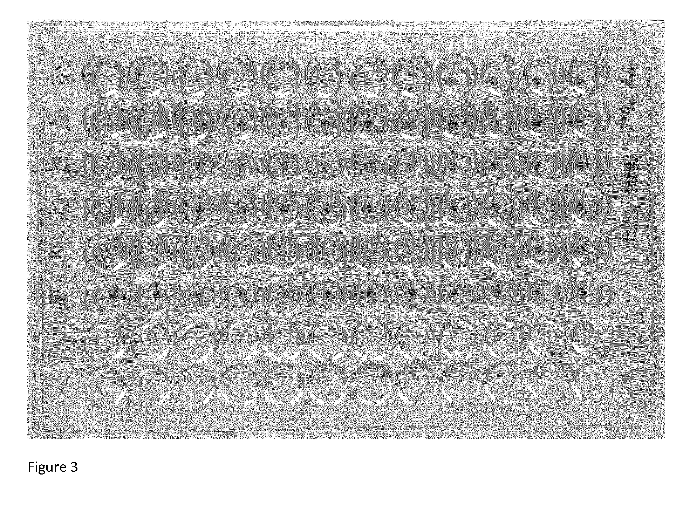

Figure 3: figure 3 shows the use of the Magnetic sulfated cellulose particles according to the present invention to concentrate MDCK cell derived Influenza A/PR/8/34 virus particles. Concentration of virus was quantified by the hemag-glutination assay whereby
   - V 1:30: virus solution from production 1:30 diluted in binding buffer
   - S1: supernatant 1^{st} washing step
   - S2: supernatant 2^{nd} washing step
   - S3: supernatant 3^{rd} washing step
   - E: Elution (desorbed virus solution).

### Detailed description of the present invention

In a first aspect, the present invention relates to a process for the preparation of magnetic sulfated cellulose particles comprising the steps of providing
- a mixture of chloro-sulfonic acid and pyridine;
- adding magnetic cellulose particles to said mixture and
- reacting the same, e.g. at a temperature of 80 or lower, like at a temperature of 70°C or lower to obtain magnetic sulfated cellulose particles.

That is, by treating the magnetic cellulose particles under the specific conditions identified herein, it is possible to provide magnetic sulfated cellulose particles allowing effective, fast and reliable isolation and purification of proteinaceous compounds including whole virus and virus proteins even with high yield from a material containing the same. This is particularly true in production processes, e.g. in bioreactors wherein the particles are recycled for further purification and isolation. The present invention is based on the finding that under specific reaction conditions, it is possible to modify the magnetic cellulose particle, i.e. to functionalize the particle to contain sulfate groups, thus, providing sulfated magnetic cellulose particles, allowing highly effective purification of whole virus and virus proteins.

As starting material, known magnetic cellulose particles may be used. For example, the magnetic cellulose particles are cellulose particles which may even contain additional components.

The sulfation of the cellulose is conducted with a sulfating agent known in the art, e.g. as described in WO 2008/125361 which is incorporated herein by reference in full. The sulfating agent includes chloro-sulfonic acid or anhydrous sulfuric acid in an organic solvent, like pyridine. The process for the preparation of magnetic sulfated cellulose particles may contain additionally the step of cooling the reaction mixture to ambient temperature after reacting the magnetic cellulose particles with the mixture of chloro-sulfonic acid and pyridine. Furthermore, the process may additionally comprise the step of neutralizing the reaction mixture with a base. Said base is preferably an aqueous sodium hydroxide solution.

In an embodiment, the process of the present invention for the preparation of magnetic sulfated cellulose particles may additionally comprise the step of washing the obtained magnetic sulfated cellulose particles with a suitable buffer, e.g. with phosphate buffered saline solution in order to provide a magnetic sulfated cellulose particles ready for use. The magnetic sulfated cellulose particles can be stored in phosphate buffered saline solution or 20% ethanol. Alternatively, the sulfated magnetic cellulose particles according to the present invention is dried for storage.

The magnetic sulfated cellulose particles according to the present invention achieved high product recoveries while reducing the level of contaminants. Furthermore, the particles are particularly useful for efficient purification of proteinaceous compounds including whole virus and virus proteins in batch and continuous processes including bioreactor systems.

In an embodiment of the present invention, the cellulose of the magnetic cellulose particle is regenerated cellulose.

The skilled person is well aware of suitable magnetic cellulose particles useful according the invention. Typically, the magnetic cellulose particle is a particle containing inorganic particle(s), like inorganic nanoparticle(s). These inorganic particles are typically responsible for the magnetic properties of the magnetic cellulose particle(s) according to the present invention. In an embodiment, the inorganic particle, in particular, the inorganic nanoparticle is chosen from magnetic metal particle. Suitable magnetic metal particles, for example are Fe₃O₄ or γ-Fe₂O₃ nanoparticles.

In an embodiment, adding chloro-sulfonic acid or anhydrous sulfuric acid into the organic solvent, like pyridine, is conducted at a temperature below or equal to 30°C, like below or equal to room temperature, like below or equal to 10°C, e.g. equal or below a temperature of 0°C. In an embodiment, reacting the mixture of chloro-sulfonic acid or anhydrous sulfuric acid in the organic solvent, like, pyridine, is conducted at a temperature of 30°C or above, preferably at a temperature of from 30°C to 120°C, for example, at a temperature of 30°C to 80°C, e.g. at 35°C or above, like at 60°C or above.

In another embodiment, after reacting the mixture of pyridine and chloro-sulfonic acid or anhydrous sulfuric acid, said mixture is cooled to a temperature of 80°C or below, to a temperature of 60°C or below, for example, at a temperature in the rage of from 40°C to 10°C, like 40°C to 20°C, in particular, 40°C to 30°C.

In another embodiment, the temperature for reacting the mixture of pyridine and chloro-sulfonic acid or anhydrous sulfuric acid with the magnetic cellulose particle is at a temperature of 120 or below, e.g. at a temperature of 80°C or below, like at a temperature of 70°C or below, as well as like at a temperature of at least 30°C,like at a temperature of at least 40°C, for example, within the range of from 30°C to 80°C, like within the range of from 30°C to 70°C, for example below 65°C, like within the range of from 50°C to 35°C to obtain the sulfated magnetic cellulose particle.

It is important that the temperature of the reaction mixture is not to high, thus, destroying the cellulose or the magnetic cellulose particle.

In an embodiment of the present invention, the process is a process comprising the steps of:
a) adding chloro-sulfonic acid to pyridine at a temperature below or equal to 30 °c;
b) reacting said mixture at a temperature of 60 °C or above;
c) optionally, cooling the mixture at a temperature of 80 °C or below;
d) adding magnetic cellulose particles to said mixture and
reacting the same at a temperature of from 30°C to 120, like at a temperature of from 40°C to 80°C to obtain magnetic sulfated cellulose particles.

In a further embodiment, magnetic sulfated cellulose particles obtainable with a method according to the present invention are provided. In an embodiment, the degree of sulfation of the cellulose of the magnetic sulfated cellulose particles according to the present invention is in the range of from 0.01% to 35% based on the cellulose. For example, the degree of sulfation of the cellulose is in the range of from 2% to 25%, e.g. 5% to 15% based on the cellulose.

The magnetic sulfated cellulose particles according to the present invention allow purifying proteinaceous compounds including whole virus or virus components, in particular, virus proteins like virus membrane proteins, virus-like particles (VLP), micelles and other nano-particles containing proteinaceous compositions.

In an embodiment of the present invention, the magnetic sulfated cellulose particles comprise cellulose, which is regenerated cellulose.

In addition, the magnetic sulfated cellulose particles according to the present invention can be separated from culture easily by using magnetic separation units known in the art. Thus, the particles according to the present invention can be easily separated from the liquid, typically, the culture medium containing cells and virus particles and, after subsequent downstream processing, including separation of virus particles and the magnetic sulfated cellulose particles, these magnetic sulfated cellulose particles can be recycled and reused for further separation cycles.

Moreover, the magnetic sulfated cellulose particles according to the present invention achieved high product recoveries and contaminant reduction. Due to a fast binding kinetics, the capturing process can be effected fast and easily and can be used in a continuous process wherein the separated magnetic sulfated cellulose particles having whole virus or virus proteins or other proteinaceous components on their surface are separated in a first step in a magnetic separation unit, eventually taken out from the system to allow separation of the whole virus or virus particles from the magnetic sulfated cellulose particles and optionally, reintroducing said particles back into the system.

Compared to other particle systems known in the art, namely, anionic beads or antibody coated beads also allowing separation by magnetic separation units, the particle according to the present invention are superior with respect to contaminant reduction and specificity to whole virus while being useful for different types of virus in contrast to antibody based systems which are typically useful for the isolation of one specific type of virus only.

That is, the advantages of the magnetic sulfated cellulose particle according to the present invention compared to the particle or bead systems known so far are:
- Bio-affinity of the sulfated cellulose is more specific than general anionic interactions for a wide range of different virus incorporating e.g. a heparin binding domain.
- Low co-adsorption of nucleic acids, as this is the case with anion exchangers, increasing the binding capacity of the particles or beads
- Different antibodies for different virus types are not needed. Therefore, the particles or beads are more versatile and cheaper than conventional antibody-coated beads.
- Simultaneous concentration of different types of virus for quantitative and qualitative characterizations. Soft desorption conditions compared to antibody based affinity particles or beads and therefore possibility of recycling and reusing the particles or beads.
- The presence of the magnetic components allow to apply a new separation method, namely magnetic based separation of the particles loaded with the proteinaceous compounds compared to beads like the cellufine beads present in columns.

In a further aspect, the present invention relates to the use of the magnetic sulfated cellulose particles according to the present invention for isolating proteinaceous compositions. In an embodiment, said proteinaceous compositions are whole virus or fragments thereof. Thus, in another embodiment, the present invention relates to the use of magnetic sulfated cellulose particles according to the present invention for the purification of compositions containing virus and/or non-virus proteins as well as virus-like particles (VLP), micelles and other nano-particles containing proteinaceous compositions. Preferably, said use leads to the isolation of whole virus including vaccinia virus and influenza virus or proteinaceous fragments thereof.

In addition, the whole virus or viral proteins or fragments thereof as mentioned herein can originate from different types of viruses, for example, from viruses of the families orthomyxoviridae, adenoviridae, herpesviridae, arenaviridae, flaviridae, retroviridae, hepadbaviridae, papovaviridae, parviviridae, poxoviridae, and the like. Preferred viruses are adeno-associated virus (AAV), cytomegalovirus (CMV), Dengue virus, Epstein-Barr virus (EBV), foot and mouth disease virus, Hepatitis B virus (HBV), Hepatitis C virus (HCV), Hepatitis E virus (HEV), human immunodeficiency virus (HIV), Herpes simplex virus (HSV), human papilloma virus (HPV), Japanese encephalitis virus (JEV), Lentivirus, respiratory synctial virus (RSV), vaccine virus, varicella zoster virus, bacteriophages T4, and F8, measles virus, lassa virus, yellow fever virus, rubella virus, vesicular stomatis virus, human immunodeficiency virus (HIV), Simian immunodeficiency virus SIV, Enterovirus 71 (EV71), baculo virus, adenovirus, Molony murine leukemia virus, and influenza virus.

For example, the virus particles and/or virus proteins or fragments originate from influenza virus including A/Puerto Rico/8/34 (H1N1), A/Wisconsin/67/2005 (H3N2), B/Malaysia/2506/2004.

In a further embodiment, the present invention relates to a method for isolating whole virus or virus proteins, preferably whole virus, comprising the step of affinity purification, in particular, pseudo-affinity purification using the magnetic sulfated cellulose particles obtainable according to the method according to the present invention or as defined herein.

Moreover, a method for isolating heparin binding molecules comprising the step of pseudo-affinity purification using the magnetic sulfated cellulose particles obtainable by a method according to the present invention or using the magnetic sulfated cellulose particles as defined herein is provided.

The method is particularly useful in high cell density cultures (HCDC) for the production and, in particular, production and purification of whole virus or viral vectors including influenza, MVA, virus-like particles (VLP), micelles and other nano-particles containing proteinaceous compositions as well as the types of virus as defined above.

Finally, the present invention relates to a bioreactor system comprising a bioreactor and a magnetic separation unit. The magnetic separation allows separating magnetic sulfated cellulose particles according to the present invention, optionally, said particle have viral components including whole virus bound thereto, from culture medium. That is, the present invention provides a bioreactor system comprising at least the components of a bioreactor and a magnetic separation unit. The magnetic sulfated cellulose particles are particularly useful for known biotechnological processes in particular, for high yield processes including cyclic or continuous cultivation and a production of virus in a bioreactor. The magnetic sulfated cellulose particles are present continuously in the bioreactor or may be added periodically to the bioreactor or are added to removed solution from the bioreactor, thus, allowing binding of the whole virus or viral fragments to the magnetic sulfated cellulose particles.

The culture medium containing the magnetic sulfated cellulose particles according to the present invention optionally with bound whole virus are transferred via suitable conduits or pipes to the magnetic separation unit where the particles are separated from the culture medium and the culture medium is optionally reintroduced into the bioreactor while the separated particle are processed further to desorb/elute the captured virus from the particles. The particles may be recycled and reused in the system accordingly. This system and the process using said system allows purifying virus during the cyclic or continuous process in the bioreactor and a continuous separation of the virus from the system. This is particularly useful in high yield culture methods since separation and eventually purification of the virus is effected while the virus is produced. Moreover, using the magnetic sulfated cellulose particles according to the present invention allows to desorb/elute and purify virus from small volumes, thus, avoiding further concentration steps which also allows saving money, time and material.

According to the present invention, the medium comprising the whole virus and/or viral proteins or fragments thereof from which capture and separation is conducted using the magnetic sulfated cellulose particles according to the present invention may be derived from: egg culture or cell culture, such as mammalian cell culture. For example, the cell culture is a cell culture using MDCK, MDBK, VERO, CV-1, MRC-5, WI-38, AGE1.CR, AGE1.CR.pIX or an LLC-MK2 cell culture.

The particle according to the present invention allows separating and purifying various types of whole virus or viral vectors as described. Thus, it is not necessary to provide expensive separation means coated with specific antibodies. In addition, in contrast to anionic beads described in the art the contaminants are reduced, in particular, proteinaceous contaminants not stemming from virus, like albumin, are reduced as well as DNA contaminants which are not desired for a high yield production process.

The magnetic sulfated cellulose particles or beads according to the present invention can be used for enrichment steps during sample preparation in diagnostic tests, in particular, in vitro diagnostic sample preparation and/or analysis, thus, allowing to decrease the limit of detection (LOD) in said in vitro diagnostic assays.

The following examples illustrate the invention further without limiting the same to the specific embodiment described in the example.

### Examples

### Preparation example

A magnetic sulfated cellulose particle is obtained as follows:
To 15 ml pyridine, chloro-sulfonic acid (1.65 ml) is added drop wise below or at 0°C. Additional 10 ml pyridine is added under stirring at ambient temperature. After the addition, the mixture is heated up to 65°C while stirring, thus, avoiding any crystals in the reaction mixture. After mixing at 65°C, the reaction mixture is cooled down to 40°C.

Separately, 200 mg of Promega Magne^{™} protein A beads (20% slurry, article number G8781) core-shell magnetic cellulose particles are washed twice with 5 ml pyridine and resuspended in 5 ml pyridine. The suspended cellulose particles in 5 ml pyridine are added to 26.65 ml pyridine-chloro-sulfonic acid reaction mixture at 40°C. The reaction mixture containing the magnetic cellulose particles is cooled to a temperature of about 35°C and reaction is continued under stirring at 35°C for 12 hours and then allowed to cool down to ambient temperature during the further process steps. After incubation, the supernatant is decanted and the particles are washed with water and phosphate buffer saline and are resuspended for further usage in phosphate buffer saline. For storage, the obtained particles are suspended in 20% ethanol in water or dried.

### Example 2:

Magnetic sulfated cellulose particles as described in Example 1 have been used to concentrate MDCK cell derived Influenza A/PR/8/34 virus particles and were quantified by the hemagglutination assay [3]. In figure 3 Evalution of magnetic sulfated cellulose particles with core shell structure is shown whereby
- V 1:30: virus solution from production 1:30 diluted in binding buffer
- S1: supernatant 1^{st} washing step
- S2: supernatant 2^{nd} washing step
- S3: supernatant 3^{rd} washing step
- E: Elution (desorbed virus solution).

MSCP method for influenza virus purification:
1. Virus suspension was diluted 1:30 in binding buffer (50mM NaCl, 10mM Tris/HCl pH 7.4).
2. MSC particles from 500µL of MSCP solution were washed 2x in binding buffer.
3. 1 mL of 1:30 virus solution was added to the washed MSCP.
4. Incubation for 10min for virus binding.
5. MSCP were washed 3x with binding buffer to remove residual unbound virus. (Sample S1-S3 was taken from the supernatant from every washing step.)
6. 100µL of desorption buffer was added and incubated for 5min.
7. 100µL of the desorbed virus solution was used for the HA-assay. First row of HA-assay starts with a 1:2 dilution, then the sample gets in every further row 1:2 diluted, which resembles a 1:2¹² = 1:4096 dilution on the most right row.

The hemagglutination assay is routinely used for the quantification of influenza virus particles. The assay is based on the interaction of hemagglutinin, a glycoprotein located on the virus surface, with sialic acids on the surface of erythrocytes. As each of the virus particles attaches to multiple erythrocytes, a lattice structure with a reduced sedimentation coefficient is formed. This phenomenon is used to quantify the virus by mixing serial dilutions of virus with a defined amount of erythrocytes. The sedimented erythrocytes are detected spectrophotometrically, and the inflection point of the read out is used for virus quantification.

In the figure 3 it can be seen, that the starting virus solution in row A (V 1:30) can be concentrated with the help of the MSCP. The eluted sample in row E shows an elevated concentration of influenza virus compared to the starting sample. In addition, in the supernatants of the washing steps, the viral titer is low. The more clear the wells from the left to the right, the higher the virus concentration is. Hence, it is clear that separation and enrichment of virus is possible with MSCPs according to the present invention.

### References

[1] A. Sakudo, K. Baba, M. Tsukamoto, A. Sugimoto, T. Okada, T. Kobayashi, N. Kawashita, T. Takagi, K. Ikuta, Bioorganic & Medicinal Chemistry 17 (2009) 752.
[2] B. Kalbfuss, M. Wolff, L. Geisler, A. Tappe, R. Wickramasinghe, V. Thom, U. Reichl, Journal of Membrane Science 299 (2007) 251.
[3] B. Kalbfuss, A. Knochlein, T. Krober, U. Reichl, Biologicals 36 (2008) 145.

## Claims

1. A process for the preparation of magnetic sulfated cellulose particles comprising the steps of providing
- a mixture of chloro-sulfonic acid and pyridine;
- adding magnetic cellulose particles to said mixture and
- reacting the same, in particular, at a temperature of 120°C or lower, like 80 or lower, to obtain a sulfated magnetic cellulose particles.

2. The process according to claim 1, wherein the cellulose of the magnetic cellulose particles is regenerated cellulose.

3. The process according to claim 1 or 2 further comprising the step of cooling the reaction mixture to ambient temperature and/or subsequently neutralizing the reaction mixture with the base.

4. The process according to any one of the preceding claims wherein the magnetic cellulose particles are particles containing inorganic particles, like inorganic nanoparticles.

5. The process according to claim 4 wherein the inorganic particles, in particular, the inorganic nanoparticles, are chosen from magnetic metal-particles, in particular, Fe₃O₄ or γ-Fe₂O₃ nanoparticles.

6. The process according to any one of the preceding claims wherein the process comprises the steps of
a) adding chloro-sulfonic acid to pyridine at a temperature below or equal to 30 °C;
b) reacting said mixture at a temperature of 60 °C or above;
c) optionally, cooling the mixture at a temperature of 80 °C or below;
d) adding magnetic cellulose particles to said mixture and
e) reacting the same at a temperature of from 30°C to 120°C, like at a temperature of 40°C to 80°C to obtain magnetic sulfated cellulose particles.

7. Magnetic sulfated cellulose particles obtainable with a method according to any one of claims 1 to 6.

8. The magnetic sulfated cellulose particles of claim 7 having a degree of sulfation of the cellulose in the range of from 0.1 to 35 % based on the cellulose.

9. The magnetic sulphated cellulose particles according to claim 7 or 8, wherein the cellulose is a regenerated cellulose.

10. A use of magnetic sulfated cellulose particles according to any one claims 7 to 9 for isolating proteinaceous compositions.

11. The use according to claim 10, wherein the proteinaceous compositions are whole virus or proteinacous compositions is a composition of virus proteins and/or non-virus proteins.

12. The use according to claim 11, wherein the proteinaceous compositions is influenza or vaccinia virus or proteinaceous fragments thereof.

13. A method for isolating whole virus or virus proteins, preferably whole virus, comprising the step of pseudo-affinity purification using magnetic sulfated cellulose particles obtainable according to any one of claims 1 to 6 or as defined in any one claims 7 to 9.

14. A method for isolating heparin binding molecules comprising the step of pseudo-affinity purification using magnetic sulfated cellulose particles obtainable according to any one of claims 1 to 6 or using magnetics sulfated cellulose particles according as defined in one of claims 7 to 9.

15. The method according to claim 13 or 14 for use in high cell density culture for the production of virus or viral vectors including Influenza, and MVA

16. A bioreactor system comprising a bioreactor and a magnetic separation unit whereby said magnetic separation unit allows to separate magnetic sulfated cellulose particles, optionally having viral components including whole virus bound thereto, from the culture.
